# EUROPEAN PATENT APPLICATION

(11) **EP 4 467 150 A1**
(43) Date of publication of application: **27.11.2024**
(21) Application number: 23178113.9
(22) Date of filing: 07.06.2023
(51) Int. Cl.: A61K 36/28, A01N 65/12

(54) **INDUSTRIAL ULTRASOUND EXTRACTION OF SESQUITERPENE LACTONES RICH-EXTRACTS**

(30) Priority: 24.05.2023 PT 2023118674
(71) Applicant: Cebal - Centro De Biotecnologia Agrícola E Agro-Alimentar Do Alentejo, 7801-908 Beja (PT)
(72) Inventor: AMOREIRA SOUSA E SILVA BRÁS, TERESA ISABEL DE, BEJA (PT); FIRMINO ROSA, DANIELA FILIPA, BEJA (PT); PEREIRA DUARTE RICARDO, MARIA DE FÁTIMA, BEJA (PT)
(74) Representative: Pereira da Cruz, Joao

(57) **Abstract**

The present disclosure is related to the extraction of sesquiterpene lactones from *Cynara cardunculus* leaves using ultrasound assisted extraction at industrial scale, and simultaneously a method to obtain sesquiterpene lactones (SLs)-enriched extracts, from leaves of *Cynara cardunculus.* In order to obtain the sesquiterpene-lactones enriched extract, the present patent application discloses steps to selectively extract SLs through industrial ultrasound assisted extraction, increasing SLs extract purity.

## Description

### Field of the Invention

The present invention is related to a method for the extraction of sesquiterpene lactones from *Cynara cardunculus* leaves using ultrasound assisted extraction at industrial scale, and simultaneously a method to obtain sesquiterpene lactones (SLs)-enriched extracts, from leaves of *Cynara cardunculus.*

In order to obtain the sesquiterpene-lactones enriched extract, the present patent application discloses the steps to selectively extract SLs applying ultrasonic energy, increasing SLs extract purity.

### Background of the Invention

Sesquiterpene lactones (SL) group comprises more than 6000 compounds, with high biological potential (Brás et al., 2023). SLs are one of the major natural compound groups, with high prevalence in the Asteraceae family. Cynara cardunculus (Asteraceae) has on its leaves a high content of SLs (Ramos *et al.,* 2013), revealing its high biological and economical potential.

Sesquiterpenes are a class of terpenes that consist of three isoprene units and often have the molecular formula C₁₅H₂₄. Sesquiterpenes may be cyclic or contain rings, including many unique combinations. Biochemical modifications such as oxidation or rearrangement produce the related sesquiterpenoids.

Sesquiterpene lactones are a common class of sesquiterpenoids that contain a lactone ring. They are found in many plants and can cause allergic reactions and toxicity if consumed excessively, particularly in grazing livestock.

Sesquiterpenes are found naturally in plants and insects, as semiochemicals, e.g. defensive agents or pheromones.

Access to SL biological potential could be achieved by solid-liquid extraction, with solvent and extraction process as key factors for a successful extraction (Brás *et al.,* 2023).

SL research has been mainly focused on compound identification, with a large range of solvents being applied, not all corresponding to the European Medicines Agency guidelines, using different conventional extraction methodologies. Moreover, there is still a lack of scientific knowledge regarding the extraction solvent and methodology effect upon SL extraction from plants, and its bioactivity, towards final applications (Brás *et* al., 2023).

Extraction plays an important role in the way to obtain SLs from vegetable sources. Previous studies regarding SLs extraction from leaves of *Cynara cardunculus* plants were focused on cynaropicrin (the major SLs present in *Cynara cardunculus* leaves) extraction by different extraction methodologies and different extraction solvents, as disclosed by Brás *et al.,* 2020 and in Patent No. EP3466936. However, the process disclosed by said patent is at laboratory scale and focused on cynaropicrin extraction yield, not being adapted for an industrial scale with a surprising impact on sesquiterpene lactones purity of the obtained extracts.

Brás *et al.,* 2020discloses that, upon cynaropicrin from cardoon leaves extraction optimization pulsed ultrasound assisted extraction (PUAE) applying a single sonotrode and ethanol with extraction solvent, allowed the best extraction yield and highest cynaropicrin concentration per g of dried biomass. However, other compounds such as monosaccharides can also be extracted, decreasing cynaropicrin and consequently sesquiterpene lactones extract purity.

Recently, Brás *et al.,* 2021 disclosed the purification of ethanolic cardoon leaves pulsed ultrasound assisted extract, by nanofiltration. In this work, a cynaropicrin enriched extract was produced using nanofiltration for the removal of low biological compounds, such as sugars. With good results in what regards to sugars removal, this purification step also increases economical costs.

### General Description of the Invention

The present disclosure relates to a novel method for the extraction of sesquiterpene lactones (SLs) present in leaves of *Cynara cardunculus.* The extraction is carried out through industrial ultrasound assisted extraction.

In the present invention, due to the type of extraction carried out, no additional extraction and/or purification steps are required, having an enormous advantage from a logistic and environmental perspective.

The present method comprises the steps of grounding air dried *Cynara cardunculus* leaves, and ultrasonic assisted extraction, wherein the obtained extract is sesquiterpene-lactone rich, and wherein ultrasonic assisted extraction is performed by an industrial ultrasound equipment.

In one embodiment, the industrial ultrasound device comprises an ultrasonic processor with power from 500 to 1000 W, and is equipped with a cascatrode usable in flow cell or batch operation. The presence of cascatrode ensures an increase of surface area, leading to an increase of extraction yield. This enables to eliminate the purification steps typically needed in these types of processes, which turns the industrial process more efficient, either from a technical point of view, but also from the environmental point of view, because it reduces the waste in the whole process and reduces the means needed to obtain a rich sesquiterpene-lactone rich extract. The use of a titanium cascatrode enhances a sesquiterpene lactone purity increase of at least 60%.

In an embodiment, the cascatrode with an approximate length between 200 and 300 mm, preferably 234mm + 10mm, with a work surface of 15 - 50 cm², input amplitude maximum of 50 micron and an amplification factor between 1:1 and 1:3, preferably 1:1.4. This ensures a proper extraction efficiency.

In one embodiment, *Cynara cardunculus* leaves are air dried and grounded until granulometry < 1000 µm.

In one embodiment the extraction solvent is ethanol 99 % v/v.

In one embodiment, extraction time is regulated by ultrasonic energy, which ranges between 30,000 and 80,000 kJ, preferably 41,500 kJ.

In one embodiment, agitation is provided by a stirring equipment with a stirring (2) shaft at 100 - 400 rpm, preferably 200 rpm.

In one embodiment, the biomass to solvent ratio (g/mL) ranges between 1/100 and 1/15, preferably between 1/60 and 1/20.

In one embodiment, liquid fraction obtained from the extraction is vacuum filtered by a filter, preferably with a pore size of less than 15 µm. This ensures that the removal of biomass and the achievement of a clean, with no solids, extract.

In an embodiment, a bioherbicide using the extract of the present invention is provided. The fact that the herbicide comprises such an extract, obtained in a surprisingly simplified way when compared to the methods of the state of the art, impacts in the higher purity and less toxicity of the bioherbicide, with has a benefit in the environment, particularly in plants subject to said bioherbicide.

In a preferred embodiment, the present disclosure encompasses a method for obtaining a sesquiterpene-lactone rich extract comprising the following steps:
grounding of air-dried *Cynara cardunculus* leaves, preferably until granulometry < 1000 µm, to obtain a biomass;
subjecting said biomass to an extraction in an ultrasonic equipment, wherein the extraction is carried out with an extraction solvent, until an extract with a solid biomass fraction to liquid fraction of solvent ratio (g/mL) from 1/15 to 1/100 is obtained;
optionally stirring the extract in a stirring equipment;
wherein the ultrasonic equipment comprises a cascatrode suitable for flow cell or batch operation, preferably a titanium cascatrode.

In a further embodiment, the present disclosure encompasses a method wherein the extraction solvent is ethanol, ethyl acetate, and ethanol/water, preferably ethanol at 99% (v/v).

In a further embodiment, the present disclosure encompasses a method further comprising the step of filtering the liquid fraction under vacuum, preferably with a filter of pore size of less than 15 µm.

In a further embodiment, the present disclosure encompasses a method wherein the ultrasonic equipment is an ultrasonic generator, optionally with power for 500 and 100W, an ultrasonic transducer, an ultrasonic cascatrode, a vacuum pump, a refrigeration bath or a combination thereof.

In a further embodiment, the present disclosure encompasses a method wherein the cascatrode comprises a length between 200 and 300 mm, preferably 234mm + 10mm and a work surface from 5 - 75 cm², preferably 15 - 50 cm². These particular measures of surface allow an adequate extraction, reducing the extraction time and preventing the degradation of the compounds.

In a further embodiment, the present disclosure encompasses a method wherein the ultrasonic equipment operates with an ultrasonic energy from 20000kJ and 90000kJ, preferably 30000kJ and 80000kJ, even more preferably preferably 41,500 kJ. These particular energy values are what will dictate the end of the extraction. It is the proper energy to obtain an increase in the purity of sesquiterpene lactones.

In a further embodiment, the present disclosure encompasses an extract obtained by the method of the present disclosure.

In a further embodiment, the present disclosure encompasses a bioherbicide comprising the extracts obtained by the method of the present disclosure.

In a further embodiment, the present disclosure encompasses a bioherbicide for use in agriculture, particularly as a phytopharmaceutical.

### Brief Description of Figures

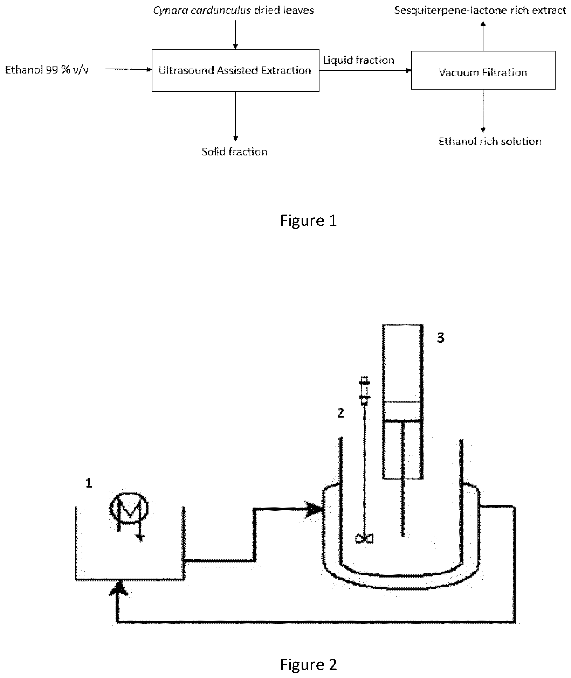

**Figure 1****.** Scheme of one embodiment of the invention.
**Figure 2****.** Overview of an embodiment of the invention. Reference numbers: 1: refrigeration bath; 2: stirring equipment; 3 - Ultrasound equipment.

### Detailed Description of the Invention

In an embodiment, for ultrasonic extraction, the following pure solvents or solvent mixtures are used as extraction solvents: ethanol, ethyl acetate, and ethanol/water. The extraction procedure aims at placing together, in a closed container protected from light, subjected to ultrasound, bath, probe or similar equipment, the lyophilised biomass and a solvent or mixture of solvents, at a particular biomass to solvent ratio, during a particular period of time, and at a particular ultrasonic power value. For this procedure, any ultrasonic wave emission device can be used, from probes to baths.

In the present invention, the extraction time is regulated by ultrasonic energy, which ranges between 30 000 and 80 000 kJ, preferably 41 500 kJ. As to biomass to solvent ratio (g/mL), it ranges between 1/100 and 1/15, preferably between 1/60 and 1/20.

The ultrasound industrial device comprises an ultrasonic processor with power from 500 to 1000 W, equipped with a cascatrode usable for flow cell or batch operation, with a work surface approximately from 15 - 50 cm², input amplitude maximum of 50 micron and an amplification factor between 1:1 and 1:3, preferably 1:1.4.

After extraction, liquid fraction is vacuum filtered by a filter, preferably with less than 15 µm pore size, and the supernatant is quantified by method and results are expressed as mg of sesquiterpene lactones/g of dry extract.

### EXAMPLES

### Example 1

Ultrasound assisted extraction, is performed with a solid-liquid ratio of 1/27 (g/mL), using ethanol 99 % v/v as extraction solvent, and ultrasonic energy of 41,500 kJ.

The apparatus includes an industrial ultrasonic processor (1 kW), 20 kHz with adjustable amplitude, a titanium cascatrode usable for flow cell or batch operation with an approximate length of 234 mm + 10 mm, an effective work surface of approximately 25 cm², input amplitude maximum of 50 micron and an amplification factor of 1:1.4.

Agitation is performed by a stirring equipment (2) with a stirring shaft at 200 rpm.

After extraction, liquid fraction is vacuum filtered by a filter, preferably with less than 15 µm pore size.

The results obtained have shown the attainment of an extract with a concentration of 473.00 m mg of sesquiterpene lactones/g of dry extract.

### Example 2

Obtaining a sesquiterpene-lactone rich extract:
Weight 148.15g of air dried Cynara cardunculus leaves
Turn the equipment UIP1000hdT on, by pressing the "on/off" button on UIP1000hdT generator.
Select "limit type" on settings screen and choose "energy". Insert the value 11.53 kWh. Go back to main menu.
After starting the experiment, perform the equiment calibration, by pressing "calibration". Always perform this step without any liquid inside the tank.
After calibration, put 4000 mL of absolute ethanol (99%) on the tank, turn on the mixer at 400rpm and at last the previously weighted leaves.
Immediately press start button, so the extraction can start.
After equipment stops, turn off the mixer and filtrate the extract using a Buchner filter of 2L and a filter paper type Whatman Nº1.
Collect the filtrated, preserve 50mL for quantifications and evaporate the remaining by using a rotary evaporator. Stored the dried extract, protected from light and air.

### Example 3

### Cynaropicrin characterization by High Pressure Liquid Chromatography (HPLC)

Cynaropicrin was quantified by HPLC. A Dionex Ultimate 3000 system (Thermo Scientific, USA), equipped with a Diode Array detector DAD-3000 (Thermo Scientific, USA) was used. A Kinetex F5 2.6µ (4.6 x 150 mm) column, from Phenomenex (USA), was used at 30ºC, with a water:acetonitrile volumetric ratio of 75:25 as mobile phase, at a flow rate of 0.5mL/min. All samples were pre-filtered with 0.22 µm pore size membrane filters (Pall, USA).

### REFERENCES

Brás, T., Neves, L. A., Crespo, J. G., & Duarte, M. F. (2020a). Effect of extraction methodologies and solvent selection upon cynaropicrin extraction from Cynara cardunculus leaves. Separation and Purification Technology, 236, 116283. https://doi.org/https://doi.org/10.1016/j.seppur.2019.116283
Brás, T., Neves, L. A., Crespo, J. G., & Duarte, M. F. (2023). Advances in sesquiterpene lactones extraction. TrAC Trends in Analytical Chemistry, 158, 116838. https://doi.org/https://doi.org/10.1016/j.trac.2022.116838
Brás, T., Paulino, A. F. C., Neves, L. A., Crespo, J. G., & Duarte, M. F. (2020b). Ultrasound assisted extraction of cynaropicrin from Cynara cardunculus leaves: Optimization using the response surface methodology and the effect of pulse mode. Industrial Crops and Products, 150, 112395. https://doi.org/https://doi.org/10.1016/j.indcrop.2020.112395
Brás, T., Rosa, D., Goncalves, A. C., Gomes, A. C., Brazinha, C., Neves, L. A., Duarte, M. F., & Crespo, J. G. (2021). Fractionation of Cynara cardunculus ethanolic extracts using diananofiltration. Separation and Purification Technology, 256, 117856. https://doi.org/https://doi.org/10.1016/j.seppur.2020.117856
Ramos, P. A. B., Guerra, A. R., Guerreiro, O., Freire, C. S. R., Silva, A. M. S., Duarte, M. F., & Silvestre, A. J. D. (2013). Lipophilic extracts of cynara cardunculus L. var. altilis (DC): A source of valuable bioactive terpenic compounds. Journal of Agricultural and Food Chemistry, 61(35), 8420-8429. https://doi.org/10.1021/if402253a

## Claims

1. Method for obtaining a sesquiterpene-lactone rich extract comprising the following steps:
grounding of air-dried *Cynara cardunculus* leaves, preferably until granulometry < 1000 µm, to obtain a biomass;
subjecting said biomass to an extraction in an ultrasonic equipment, wherein the extraction is carried out with an extraction solvent, until an extract with a solid biomass fraction to liquid fraction of solvent ratio (g/mL) from 1/15 to 1/100 is obtained and wherein the ultrasonic equipment operates with ultrasonic energy from 20000kJ and 90000kJ, preferably 30000kJ and 80000kJ, even more preferably preferably 41,500 kJ;
wherein the ultrasonic equipment comprises a cascatrode suitable for flow cell or batch operation, preferably a titanium cascatrode.

2. Method according to the previous claim wherein the extraction solvent is ethanol, ethyl acetate, and ethanol/water, preferably ethanol at 99% (v/v).

3. Method according to any of the previous claims further comprising the step of filtering the liquid fraction under vacuum, preferably with a filter of pore size of less than 15 µm.

4. Method according to the previous claim wherein the ultrasonic equipment is an ultrasonic generator, optionally with power for 500 and 100W, an ultrasonic transducer, an ultrasonic cascatrode, a vacuum pump, a refrigeration bath or a combination thereof.

5. Method according to any of the previous claims wherein the cascatrode comprises a length between 200 and 300 mm, preferably 234mm + 10mm and a work surface from 5-75 cm², preferably 15 - 50 cm².

6. Method according to any of the previous claims further comprising the step of stirring the extract in a stirring equipment.

7. Extract obtained by the method of claims 1 - 6 for use in medicine.

8. Bioherbicide comprising the extracts obtained by the method of claims 1-6.

9. Bioherbicide according to the previous claim for use in agriculture, particularly as a phytopharmaceutical.
